(19) Europäisches Patentamt
European Patent Office
Office européen des brevets

(11) **EP 4 421 655 A1**

(12) **EUROPEAN PATENT APPLICATION**
published in accordance with Art. 153(4) EPC

(43) Date of publication:
**28.08.2024   Bulletin 2024/35**

(21) Application number: **23806553.6**

(22) Date of filing: **27.02.2023**

(51) International Patent Classification (IPC):
**G06F 16/35** (2019.01)

(52) Cooperative Patent Classification (CPC):
**G06F 16/35; G06N 3/042; G06N 3/0455;
G06N 3/0464; G06N 3/098; G16C 20/20;
G16C 20/70**

(86) International application number:
**PCT/CN2023/078322**

(87) International publication number:
**WO 2023/221592 (23.11.2023 Gazette 2023/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.05.2022   CN 202210558493**

(71) Applicant: **Tencent Technology (Shenzhen)
Company Limited
Shenzhen, Guangdong, 518057 (CN)**

(72) Inventors:
• **WU, Zhenxing
  Shenzhen, Guangdong 518057 (CN)**
• **HSIEH, Chang-Yu
  Shenzhen, Guangdong 518057 (CN)**
• **ZHANG, Shengyu
  Shenzhen, Guangdong 518057 (CN)**
• **HOU, Tingjun
  Shenzhen, Guangdong 518057 (CN)**

(74) Representative: **Reddie & Grose LLP
The White Chapel Building
10 Whitechapel High Street
London E1 8QS (GB)**

(54) **CO-TRAINING METHOD FOR MODELS, AND RELATED APPARATUS**

(57)     Provided in the embodiments of the present disclosure are a co-training method and apparatus for models, and a computer program product and a storage medium. The method comprises: for each molecular representation among a plurality of molecular representations, determining a neural network model corresponding to the molecular representation; for each molecular representation, determining, by means of the neural network model corresponding thereto, a predicted molecular property result and a predicted confidence degree, which correspond to unlabeled data in a set of unlabeled data; acquiring unlabeled data, the corresponding predicted confidence degree of which is higher than a preset threshold value, and taking same as reference unlabeled data; on the basis of the reference unlabeled data and a predicted molecular property result corresponding thereto, determining pseudo-labeled data of neural network models corresponding to other molecular representations; and on the basis of the pseudo-labeled data of the neural network models, which respectively correspond to the plurality of molecular representations, performing co-training on the neural network models, which respectively correspond to the plurality of molecular representations, performing co-training on the neural network models, which respectively correspond to the plurality of molecular representations. The method improves the molecular property prediction capability of a neural network model.

FIG. 3

**Description**

RELATED APPLICATION

This application claims priority to Chinese Patent Application No. 2022105584935, filed with the China National Intellectual Property Administration on May 20, 2022 and entitled "CO-TRAINING METHOD FOR MODELS, AND RELATED APPARATUS".

FIELD OF THE TECHNOLOGY

The present disclosure relates to the field of artificial intelligence, and in particular, to a model cooperative training technology and a molecular property prediction technology based on a neural network model.

BACKGROUND OF THE DISCLOSURE

Molecular property prediction is one of the most important tasks in a process of computer-aided drug discovery. The main objective of the molecular property prediction is to predict molecular physical and chemical properties based on internal molecular information such as atomic coordinates, to assist related technicians in quickly finding compounds that meet expected properties in a large quantity of candidate compounds, and accelerate a speed of drug screening and drug design.

Currently, in the molecular property predicting process, common methods include a molecular descriptor-based method (Descriptor-based method), a molecular graph-based method (Graph-based method), a simplified molecular input line entry system (SMILES) string-based method (SMILES-based method). The molecular descriptor-based method in which a molecule is represented by using a fixed length feature vector of a predefined molecular descriptor/fingerprint is the most popular method in molecular property prediction. The molecular graph-based method describes a molecule as a molecular graph having nodes (atoms) and edges (bonds), rather than a fixed length feature vector. By using a graph neural network (GNN) framework, a molecular feature is automatically extracted from simple initial features defined based on atoms, bond features and a molecular topological structure, to perform molecular property prediction. The SMILES string-based method extracts molecular features directly from a SMILES string to perform molecular property prediction without relying on any hand-generated features.

However, the molecular descriptor-based method is sensitive to a molecular feature. The molecular graph-based method relies heavily on training data volume, and the SMILES string-based method has high requirements on the ability of feature extraction and the training data volume. It can be learned that currently, an important research direction in the field of molecular property prediction is optimize the existing molecular predicting methods and improve a molecular property prediction capability of a model.

SUMMARY

To resolve the above problems, the present disclosure provides a model co-training method and apparatus, a computer program product, and a storage medium, and a molecular property predicting method and apparatus, a computer program product, and a storage medium, to effectively improve a molecular property predicting capability.

The model co-training method provided in the present disclosure is executable by a computer device, and includes:

determining, for each of a plurality of molecular representations, a neural network model corresponding to the molecular representation, the neural network model being configured to determine, based on the molecular representation, a molecular property prediction result and a prediction confidence of the molecular property prediction result;

processing, by using the neural network model corresponding to each of the plurality of molecular representations, unlabeled data in an unlabeled data set to obtain a molecular property prediction result and a prediction confidence for the unlabeled data; and obtaining unlabeled data having a prediction confidence higher than a preset threshold as reference unlabeled data, and determining, based on the reference unlabeled data and a molecular property prediction result corresponding to the reference unlabeled data, pseudo-labeled data for a neural network model corresponding to another molecular representation in the plurality of molecular representations; and

performing, based on pseudo-labeled data for the neural network models corresponding to the plurality of molecular representations, co-training on the neural network models corresponding to the plurality of molecular representations, to obtain trained neural network models for predicting a molecular property.

An embodiment of the present disclosure further provides a molecular property predicting method, executable by a computer device, the method including:

obtaining a molecular representation of a to-be-predicted molecule;

performing property prediction on the to-be-predicted molecule based on the molecular representation by using a neural network model corresponding to the molecular representation; and

determining, based on output of the neural network model corresponding to the molecular representation, a prediction result corresponding to the to-be-predicted molecule,

the neural network model corresponding to the molecular representation being trained based on pseudo-labeled data corresponding to the neural network model, the pseudo-labeled data corresponding to the neural network model comprising reference unlabeled data and a molecular property prediction result corresponding to the reference unlabeled data, the reference unlabeled data in an unlabeled data set and having a prediction confidence higher than a preset threshold, and the prediction confidence and the molecular property prediction result corresponding to the unlabeled data being determined by using a neural network model corresponding to another molecular representation.

An embodiment of the present disclosure provides a model co-training apparatus, including:

a model constructing module, configured to determine, for each of a plurality of molecular representations, a neural network model corresponding to the molecular representation, the neural network model being configured to determine, based on the molecular representation, a molecular property prediction result and a prediction confidence of the molecular property prediction result;

a training data set determining module, configured to: process, by using the neural network model corresponding to each of the plurality of molecular representations, unlabeled data in an unlabeled data set to obtain a molecular property prediction result and a prediction confidence for the unlabeled data; and obtain unlabeled data having corresponding prediction confidence higher than a preset threshold as reference unlabeled data, and determine, based on the reference unlabeled data and a molecular property prediction result corresponding to the reference unlabeled data, pseudo-labeled data for a neural network model corresponding to another molecular representation in the plurality of molecular representations; and

a co-training module, configured to perform, based on pseudo-labeled data for the neural network models corresponding to the plurality of molecular representations, co-training on the neural network models corresponding to the plurality of molecular representations, to obtain trained neural network models for predicting a molecular property.

An embodiment of the present disclosure further provides a molecular property predicting apparatus, including:

a molecule obtaining module, configured to obtain a molecular representation of a to-be-predicted molecule;

a molecular property predicting module, configured to perform, based on the molecular representation by using a neural network model corresponding to the molecular representation, property prediction on the to-be-predicted molecule; and

a prediction result output module, configured to determine, based on output of the neural network model corresponding to the molecular representation, a prediction result corresponding to the to-be-predicted molecule,

the neural network model corresponding to the molecular representation being trained based on pseudo-labeled data corresponding to the neural network model, the pseudo-labeled data corresponding to the neural network model comprising reference unlabeled data and a molecular property prediction result corresponding to the reference unlabeled data, the reference unlabeled data being in an unlabeled data set and having a prediction confidence higher than a preset threshold, and the prediction confidence and the molecular property prediction result corresponding to the unlabeled data being determined by using a neural network model corresponding to another molecular representation.

An embodiment of the present disclosure provides a computer device, including a processor and a memory, the memory having program instructions stored therein; and the processor being configured to execute the program instructions stored in the memory, to perform the foregoing method.

An embodiment of the present disclosure provides a computer program product, including computer software code, the computer software code, when run by a processor, providing the foregoing method.

An embodiment of the present disclosure provides a computer-readable storage medium, having computer-executable instructions stored thereon, the instructions, when executed by a processor, providing the foregoing method.

The model co-training method in the present disclosure can perform cooperative modeling and co-training on a neural network model configured to predict a molecular property from different perspectives. In a co-training process, for each molecular representation, a molecular property prediction result and prediction confidence corresponding to unlabeled data in an unlabeled data set are determined by using the neural network model corresponding to the molecular representation. Then, unlabeled data having corresponding prediction confidence higher than a preset threshold is obtained as reference unlabeled data. Pseudo-labeled data for a neural network model corresponding to another molecular representation is determined based on the reference unlabeled data and a molecular property prediction result corresponding to the reference unlabeled data. Labeled data and a large quantity of pieces of unlabeled data are fully used to construct a high-precision molecular property predicting model, to overcome problems of scarce label data and a poor model training effect in a molecular property predicting process. Therefore, in the model co-training method in the present disclosure, advantages of the molecular representations are fully used, so that a predicting capability of the neural network model is improve.

BRIEF DESCRIPTION OF THE DRAWINGS

FIG. 1 is a schematic diagram of a basic process of molecular druggability prediction according to an embodiment of the present disclosure.

FIG. 2A is a schematic diagram of a molecular representation according to an embodiment of the present disclosure.

FIG. 2B is a schematic diagram of another molecular representation according to an embodiment of the present disclosure.

FIG. 2C is a schematic diagram of still another molecular representation according to an embodiment of the present disclosure.

FIG. 3 is a schematic diagram of a determining process of pseudo-labeled data when co-training is performed on a neural network model according to an embodiment of the present disclosure.

FIG. 4 is a schematic diagram of a processing process of an RGCN-based neural network model according to an embodiment of the present disclosure.

FIG. 5 is a schematic diagram of a processing process of a DNN-based neural network model according to an embodiment of the present disclosure.

FIG. 6A is a schematic diagram of a processing process of a K-BERT-based neural network model according to an embodiment of the present disclosure.

FIG. 6B is a schematic diagram of another processing process of a K-BERT-based neural network model according to an embodiment of the present disclosure.

FIG. 6C is a schematic diagram of still another processing process of a K-BERT-based neural network model according to an embodiment of the present disclosure.

FIG. 6D is a schematic diagram of yet another processing process of a K-BERT-based neural network model according to an embodiment of the present disclosure.

FIG. 7 is a schematic flowchart of a model co-training method according to an embodiment of the present disclosure.

FIG. 8 is a schematic flowchart of a molecular property predicting method according to an embodiment of the present disclosure.

FIG. 9 is a schematic diagram of a processing process based on a neural network model according to an embodiment of the present disclosure.

FIG. 10 is a schematic composition diagram of a model co-training apparatus according to an embodiment of the present disclosure.

FIG. 11 is a schematic composition diagram of a molecular property predicting apparatus according to an embodiment of the present disclosure.

FIG. 12 is an architecture of a computing device according to an embodiment of the present disclosure.

FIG. 13 is a schematic diagram of a storage medium according to an embodiment of the present disclosure.

DESCRIPTION OF EMBODIMENTS

To enable the objects, technical solutions and advantages of the present disclosure more apparent, exemplary embodiments according to the present disclosure is described in detail below with reference to the accompanying drawings. Apparently, the described embodiments are merely some but not all of the embodiments of the present disclosure. It is to be understood that the present disclosure is not limited by the exemplary embodiments described herein.

In addition, in the specification and accompanying drawings, the same or similar steps and elements are denoted by the same or similar reference numerals, and repeated descriptions of these steps and elements is omitted.

In addition, in the specification and accompanying drawings, according to the embodiments, elements are described in the singular or plural forms. However, the singular and plural forms are appropriately selected to be used in the described circumstances merely for convenience of explanation and are not intended to limit the present disclosure thereto. Therefore, the singular form may include the plural form, and the plural form may also include the singular form unless the context clearly dictates otherwise.

In the specification and accompanying drawings, the same or similar steps and elements are denoted by the same or similar reference numerals, and repeated descriptions of these steps and elements is omitted. In addition, in descriptions of the present disclosure, terms such as "first" and "second" are only used for distinguishing between descriptions and cannot be understood as indicating or implying relative importance or a sequence.

To facilitate describing the present disclosure, concepts related to the present disclosure are described below.

The method in the present disclosure may be based on the artificial intelligence (AI) technology. For example, for a method based on the artificial intelligence, machine learning may be performed in the method in a manner similar to human perception, for example, to predict molecular physical and chemical properties by training a neural network, to assist related technicians in quickly finding compounds that meet expected properties in a large quantity of compounds.

In conclusion, the solutions provided in the embodiments of the present disclosure relate to computer technologies such as artificial intelligence and a neural network. The embodiments of the present disclosure are described below with reference to the accompanying drawings.

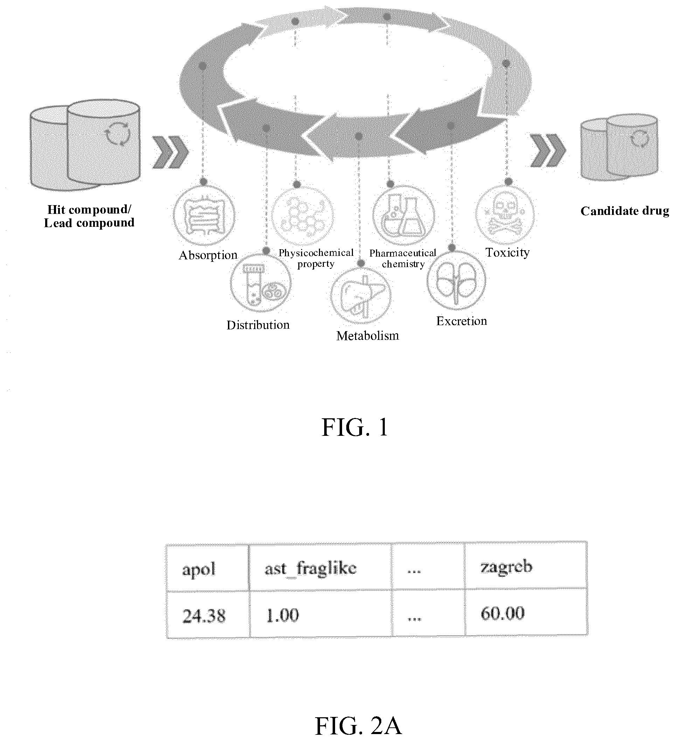

FIG. 1 is a schematic diagram of a basic process of molecular druggability prediction according to an embodiment of the present disclosure.

As shown in FIG. 1, in a process of molecular druggability prediction, verification in aspects such as absorption, distribution, metabolism, excretion, toxicity, a physicochemical property, and a pharmacochemical property is performed on pre-obtained hit compounds or lead compounds, to obtain final candidate drugs. ADMET (absorption, distribution, metabolism, excretion and toxicity of drugs) pharmacokinetics method is an important method in current drug design and drug screening. A current ADMET druggability predicting model is generally implemented based on a molecular descriptor-based molecular representation, a molecular graph-based molecular representation, or a SMILES string-based molecular representation. However, because the model constructed based on the molecular descriptor-based molecular representation, the molecular graph-based molecular representation, or the SMILES string-based molecular representation has a limitation, there are large deviations in prediction and evaluate of druggability of active molecules in an early stage of drug research and development, resulting failure of most active molecules discovered in large-scale screening in a pre-clinical research stage. Therefore, the present disclosure provides a model co-training method. According to the method, for each molecular representation, a molecular property prediction result and prediction confidence of unlabeled data is determined by using a neural network model corresponding to the molecular representation, then unlabeled data having corresponding prediction confidence higher than a preset threshold is selected to determine pseudo-labeled data for a neural network model corresponding to another molecular representation, to iteratively update network parameters of neural network models by using pseudo-labeled data for the neural network models, so that a high-precision ADMET druggability predicting model is constructed, thereby increase a research and development rate of new drugs and reducing costs of the research and development.

Verification processes of aspects such as absorption, distribution, metabolism, excretion, toxicity, a physicochemical property, and a pharmacochemical property in FIG. 1 do not have a strict order. These processes may be performed in parallel or in an order of different permutations and combinations.

FIG. 1 only shows an embodiment of molecular property prediction being applied in the molecular druggability prediction. Actually, the molecular property predicting method in the present disclosure may be applied in various molecular property prediction scenarios such as physical property prediction and chemical property prediction of molecules.

FIG. 2A is a schematic diagram of a molecular descriptor-based molecular representation according to an embodiment of the present disclosure.

A molecular descriptor is a molecular representing manner (such as a onedimensional vector shown in FIG. 2A) to describe a molecule based on a series of physicalchemical properties of a molecule or a molecular fragment structure. In the molecular descriptor-based molecular property predicting method, a fixed length feature vector of a predefined molecular descriptor/fingerprint to represent a molecule, and the method is the most popular method in molecular property prediction. In the molecular descriptor-based molecular property predicting method, molecular property modeling is performed by using a conventional machine learning (ML) method (such as a random forest, a support vector machine, and XGBoost) and combining the molecular descriptor. With continuous innovation of ML algorithms and increasing of molecular descriptors, many molecular descriptor-based models are established to evaluate potential of a specific molecule to become a drug.

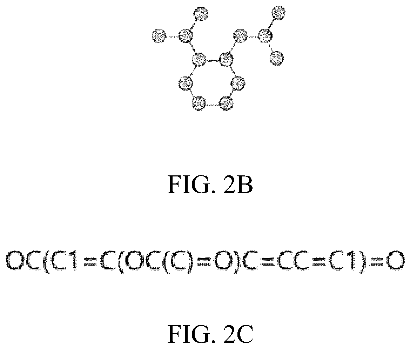

FIG. 2B is a schematic diagram of a molecular graph-based molecular representation according to an embodiment of the present disclosure.

A molecular graph is a molecule representing manner to describe a molecule as having nodes (atoms) and edges (bond). In recent year, the graph-based molecule predicting methods receive increasing attention, and addresses limitations of the molecular descriptor-based method. Different from the molecular descriptor-based method, a molecular graph-based method describes a molecule as a molecular graph having nodes (atoms) and edges (bonds), rather than a fixed length feature vector. By using a graph neural network (GNN) framework, a molecular feature is automatically extracted from simple initial features defined based on the atoms, bond features and a molecular topological structure, to perform molecular property prediction. A molecular graph-based model may automatically learn representations for atoms in a molecule in a specific task, and avoid to some extent loss of relevant information caused by manual extraction of a molecular descriptor/fingerprint.

FIG. 2C is a schematic diagram of a SMILES string-based molecular representation according to an embodiment of the present disclosure.

The simplified molecular input line entry system (SMILES) string is a simplified chemical language used for representing a molecule. Application of a SMILES-based molecular property predicting method in molecular property prediction is not popular as the molecular descriptor-based method and the molecular graph-based method. The SMILES-based molecular property predicting method may be regarded as a natural language processing method. An inherent advantage of the method is that a molecular feature can be extracted directly from a SMILES without relying on any manually generated features.

For the molecular representations shown in FIG. 2A to FIG. 2C, the molecular descriptor-based model is sensitive to a molecular feature, and generation of a molecular descriptor/fingerprint requires extensive human expert knowledge, which is a restricting factor for development of the method. The molecular graph-based model may automatically learn representations for atoms in a molecule in a specific task, and avoid to some extent loss of relevant information caused by manual extraction of a molecular descriptor/fingerprint. However, the molecular graph-based method relies heavily on training data volume, and performance of the molecular graph-based method is even inferior to the molecular descriptor-based method when a size of a training data set is small. In addition, a problem of over-smoothing is prone to occur in a graph convolution network, and in this case, a quantity of layers in the graph convolution network is generally only two to four, which limits a feature extraction capability of the graph convolution network. Therefore, the molecular graph-based method achieves excellent results on some tasks, but does not make breakthroughs in drug discovery. An inherent advantage of the SMILES-based method is that a molecular feature can be extracted directly from a SMILES without relying on any manually generated features. Because a structure and chemical information a molecule are implicit in the SMILES and are not as clear as the molecular descriptor/fingerprint and the molecular graph, the SMILES-based method have higher requirements for a feature extraction capability and training data volume. This results in that performance of the SMILES-based method in predicting molecular property may be inferior to the molecular graph-based method and the molecular descriptor-based method, and consequently, the SMILES-based method is less popular than the previous two methods. To collaboratively construct a high-precision model from different perspectives by properly using different types of methods, the present disclosure provides a model co-training method.

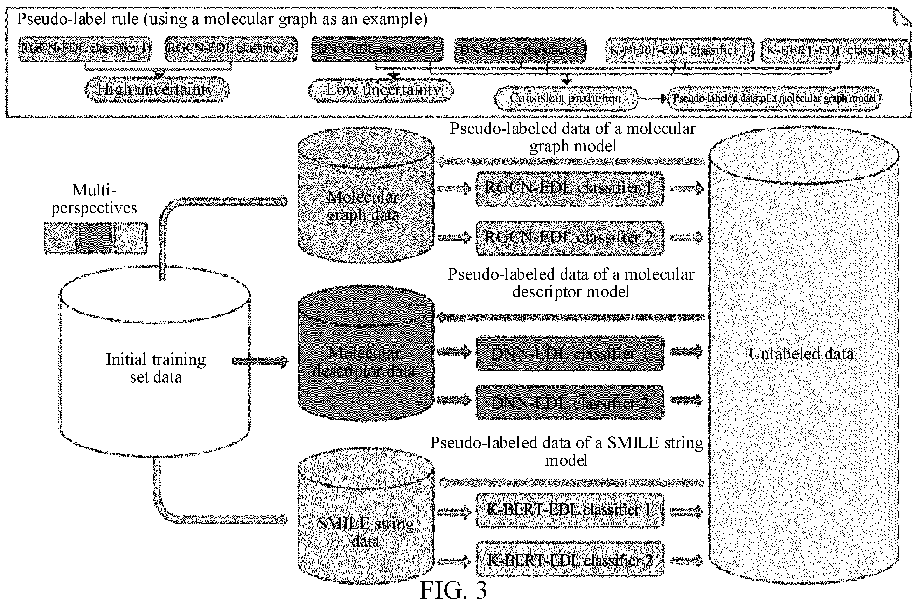

According to the embodiments of the present disclosure, a determining process of pseudo-labeled data used when co-training is performed on a neural network model is shown in FIG. 3.

According to embodiments of the present disclosure, modeling may be separately performed from three perspectives of a molecular descriptor-based molecular representation, a molecular graph-based molecular representation, and a SMILES string-based molecular representation, to perform, from different perspectives, cooperative modeling and co-training on a neural network model configured to predict a molecular property, so as to improve a molecular property predicting capability of the neural network model.

As shown in FIG. 3, for the molecular descriptor-based molecular representation, a neural network model based on a deep neural network (DNN) (that is, a DNN-EDL classifier) may be constructed. For the molecular graph-based molecular representation, a neural network model based on a relational graph convolution network (RGCN) (that is, an RGCN-EDL classifier) is constructed. For the SMILES string-based molecular representation, a neural network model based on a knowledge-based bidirectional encoder representation from transformers (K-BERT) (that is, a K-BERT-EDL classifier) is constructed. To increase variety of molecular property predicting models, two neural network models are constructed for each molecular representation based on different random seeds.

By using each constructed neural network model, initial training set data may be predicted, to obtain a prediction value and uncertainty corresponding to the prediction value. Because selected models herein are all neural network models, an evidential deep learning (EDL) method is used to evaluate uncertainty of prediction by different neural network models. When being applied, the EDL mainly changes a loss function of a neural network, so that the EDL can be easily applied to different types of neural network models. The EDL allows a model to obey Dirichlet distribution when predicting classification probability. In addition, when optimizing a loss function, the EDL reduces variance while reducing prediction loss, so that each neural network model can provide prediction uncertainty when outputting a prediction value.

Pseudo labels may be assigned to some unlabeled data based on the prediction value of each neural network model and the uncertainty corresponding to the prediction value, and a most uncertain molecule (a molecule having the largest amount of information for the model) of the model is added into a training set, and re-train the model, to further improve prediction precision of the model.

Using the RGCN-based neural network model constructed based on the molecular graph-based molecular representation as an example, molecule data that has high uncertainty (that is, low confidence) when predicted based on the RGCN-based neural network model but is correctly predicted and has low uncertainty (that is, high confidence) when predicted based on the DNN-based neural network model and the K-BERT-based neural network model may be used as pseudo-labeled data, and the pseudo-labeled data is used to train the RGCN-based neural network model, to improve a prediction capability of the neural network model.

For the DNN-based neural network model constructed based on the molecular descriptor-based molecular representation and the K-BERT-based neural network model constructed based on the SMILES string-based molecular representation, similar methods may also be used to obtain pseudo-labeled data. To be specific, for each molecular representation in a plurality of molecular representations, a molecular property prediction result and prediction confidence corresponding to unlabeled data may be determined by using a neural network model corresponding to the molecular representation, and unlabeled data having corresponding prediction confidence higher than a preset threshold is selected as pseudo-labeled data for a neural network model corresponding to another molecular representation.

Herein, unlabeled data processed by a neural network model may be classified based on a preset threshold. Unlabeled data having corresponding prediction confidence greater than or equal to the preset threshold is determined as data having high confidence, and unlabeled data having corresponding prediction confidence less than the preset threshold is determined as data having low confidence. The preset threshold herein may be determined by average uncertainty of correctly classified molecules in a verification set. For example, unlabeled data having corresponding prediction confidence greater than or equal to the average uncertainty of the correctly classified molecules in the verification set is determined as data having high confidence, and unlabeled data having corresponding prediction confidence less than the average uncertainty of the correctly classified molecules in the verification set is determined as data having low confidence.

The following further describes processing processes of the neural network models based on examples shown in FIG. 3.

A relational graph convolution network (RGCN) is an expansion of a graph convolution network, and the relational graph convolution network achieves excellent performance in node prediction and relationship prediction. In the present disclosure, the relational graph convolution network is applied in full image prediction. An information transfer rule of the relational graph convolution network is as follows:

$$h_v^{(l+1)} = \sigma \left( \sum_{r \in R} \sum_{u \in N_v^r} W_r^{(l)} h_u^{(l)} + W_o^{(l)} h_v^{(l)} \right)$$

$h_v^{(l+1)}$ is a node vector (an atomic representation) of a node v after $l+1$ iterations, $N_v^r$ is an adjacent node of the node v in a case that an edge is $r \in R$, $W_r^{(l)}$ is a weight of a node u connected to the node via the edge $r \in R$, and $W_o^{(l)}$ is a weight of the target node v. In can be learned that information of a chemical bond in the RGCN is clearly represented in a form of a relation $r \in R$. To be specific, a feature vector of each atom in the RGCN is iteratively determined based on feature vectors of surrounding atoms.

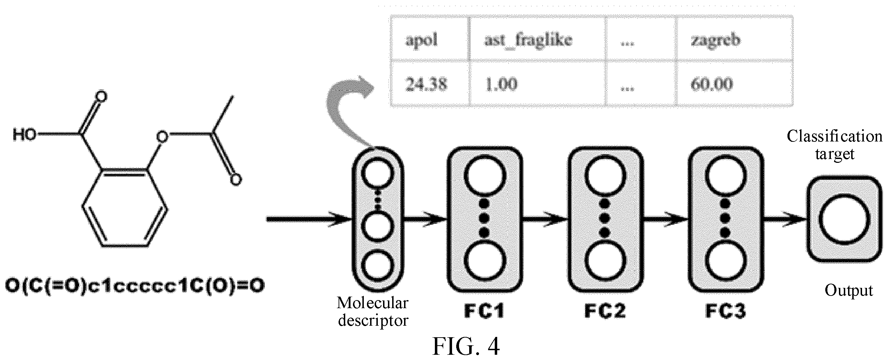

According to the embodiments of the present disclosure, a processing process of an RGCN-based neural network model constructed based on a molecular graph-based molecular representation is shown in FIG. 4.

A RGCN-based second neural network model constructed based on the molecular graph-based molecular representation includes a plurality of RGCN layers and a plurality of FC layers (using two RGCN layers and three FC layers an example herein). After a molecular graph is input into the second neural network model, the molecular graph is processed via the plurality of RGCN layers in the second neural network model, to obtain feature vectors of atoms in the molecular graph. A molecular feature vector corresponding to the molecular graph may be determined based on the feature vectors of the atoms and weights corresponding to the feature vectors of the atoms. Then, the molecular feature is processed via the plurality of FC layers in the second neural network model, to obtain a second molecular property prediction result (that is, a prediction result classified by an RGCN-EDL classifier) processed by the RGCN-based second neural network model. Aspirin is used as an example in FIG. 4. A molecular graph of the aspirin is input into the RGCN-based second neural network model including the plurality of RGCN layers and the plurality of FC layers, to obtain a result of classifying the aspirin.

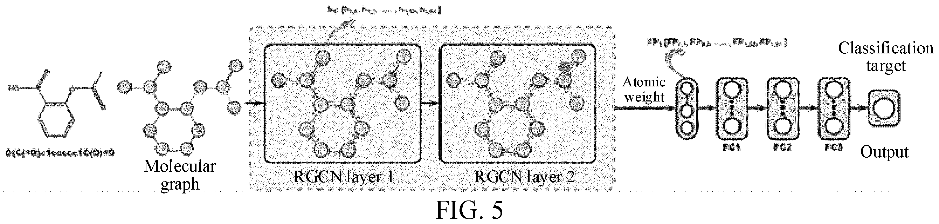

According to the embodiments of the present disclosure, a processing process of a DNN-based neural network model constructed based on a molecular descriptor-based molecular representation is shown in FIG. 5.

A DNN-based first neural network model constructed based on the molecular descriptor-based molecular representation includes a plurality of FC layers (using three FC layers as an example herein). Because the molecular descriptor-based molecular representation may be processed as a feature vector corresponding to a molecule, a molecular descriptor-based molecular feature vector may be directly input into the DNN-based first evidential neural network model, to obtain a first molecular property prediction result (that is, a prediction result classified by a DNN-EDL classifier) processed by the DNN-based first neural network model. Aspirin is as an example in FIG. 5. A molecular feature vector corresponding to a molecular descriptor of the aspirin is input into the DNN-based first neural network model including the plurality of FC layers, to obtain a result of classifying the aspirin.

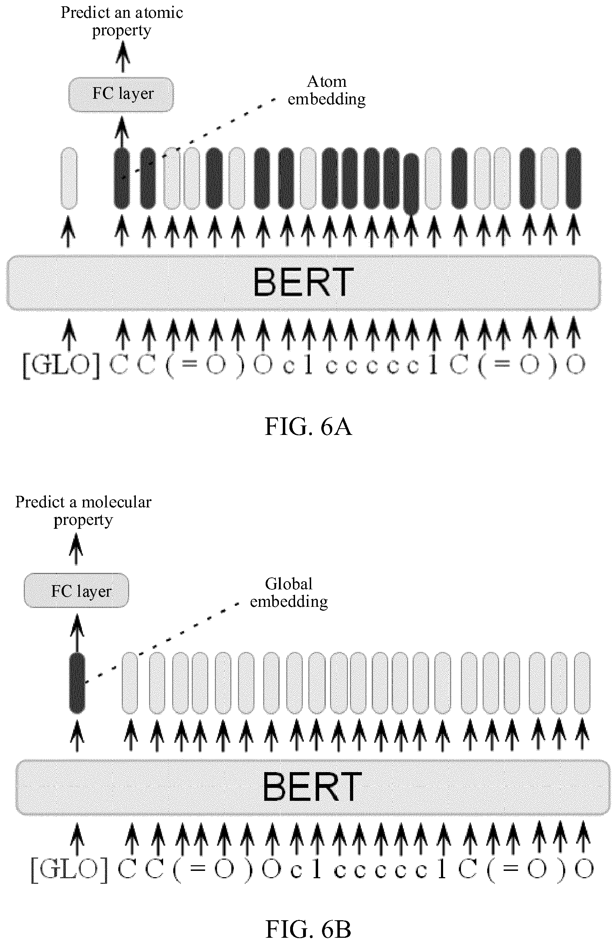

FIG. 6A to FIG. 6D are schematic diagrams of processing processes of a K-BERT-based neural network model constructed based on a SMILES string-based molecular representation according to embodiments of the present disclosure.

According to the embodiments of the present disclosure, a K-BERT-based third neural network model may have a plurality of pre-training tasks.

For example, FIG. 6A is a schematic diagram of a processing process of an atomic feature prediction and pre-training task.

In the processing process of the atomic feature prediction and pre-training task, the K-BERT-based third neural network model may learn and train an atomic feature based on pre-obtained atomic property training set data. In some embodiments, the pre-obtained atomic property training set data may be obtained by using an RDKit software to calculate an atomic feature of each heavy atom in a molecule, and an atomic property may include atomicity, aromaticity, hydrogen, chirality, a chiral type, and the like.

In a process of learning and training the atomic feature based on the K-BERT-based third neural network model, a word vector corresponding to a SMILES string-based molecular representation may be input into the K-BERT-based third neural network model, to obtain an atomic property prediction result. As shown in FIG. 6A, during atom embedding, a dark-colored part in the figure represents an atomic feature, and a light-colored part represents a feature of another structure such as a chemical bond. In the processing process of the atomic feature prediction and pre-training task, only a prediction result of the dark-colored part in the figure is concerned. Because each molecule is formed by a plurality of atoms, the atomic feature prediction and pre-training task may be regarded as a multi-task classification task.

FIG. 6B is a schematic diagram of a processing process of a molecular feature prediction and pre-training task.

In the processing process of the molecular feature prediction and pre-training task, the K-BERT-based third neural network model may learn and train a molecular feature based on pre-obtained molecular property training set data. In some embodiments, the pre-obtained molecular property training set data may be obtained by using an RDKit software to calculate a global feature of a molecule.

In a process of learning and training the molecular feature based on the K-BERT-based third neural network model, a word vector corresponding to a SMILES string-based molecular representation may be input into a K-BERT-based evidential neural network model, to obtain a molecular property prediction result. As shown in FIG. 6B, during global embedding, a dark-colored part in the figure represents a molecular feature, and a light-colored part represents a feature of another structure. In the processing process of the molecular feature prediction and pre-training task, only a prediction result of the dark-colored part in the figure is concerned. Because the global feature of the molecule may be represented by using a MACCS fingerprint, a global feature prediction task may be regarded as a multi-task classification task.

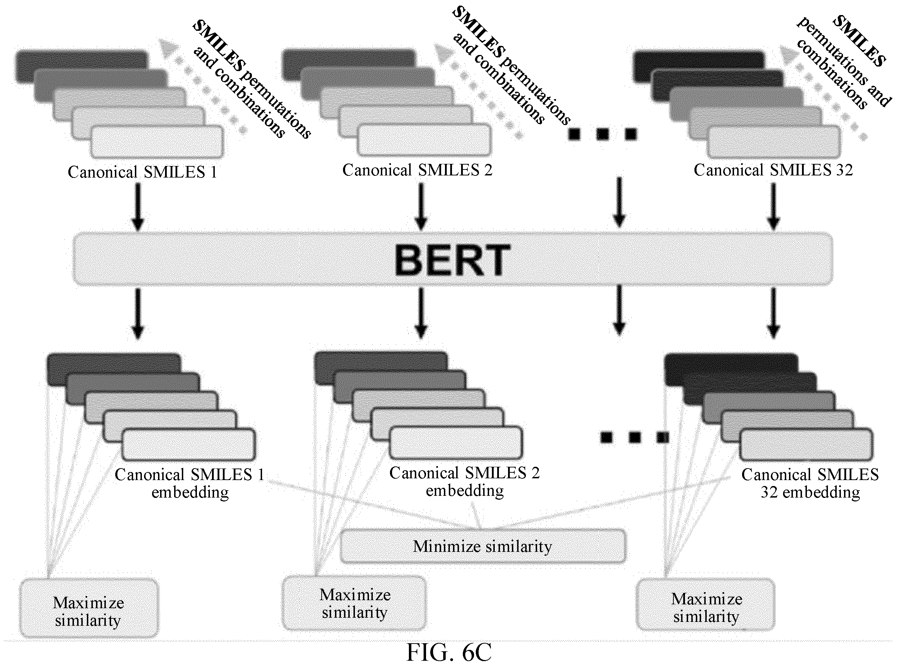

FIG. 6C is a schematic diagram of a processing process of a contrastive learning and pre-training task.

To increase variety of samples to correctively identifying and processing different SMILES strings of a same molecule, a plurality of different SMILES strings may be generated for canonical SMILES string input through SMILES permutations and combinations (for example, in FIG. 6C, four different SMILES strings are generated for each canonical SMILES string through permutation and combinations, to form a group of SMILES strings represented by different colors and shades in FIG. 6C). An objective of the contrastive learning and pre-training task is to maximize cosine similarity between embeddings of different SMILES strings of a same molecule, and minimize similarity between embeddings in different molecules, to be specific, to enable molecular property prediction results of different SMILES strings of a same molecule to be the same as much as possible, and to enable molecular property prediction results of SMILES strings of different molecules to be different.

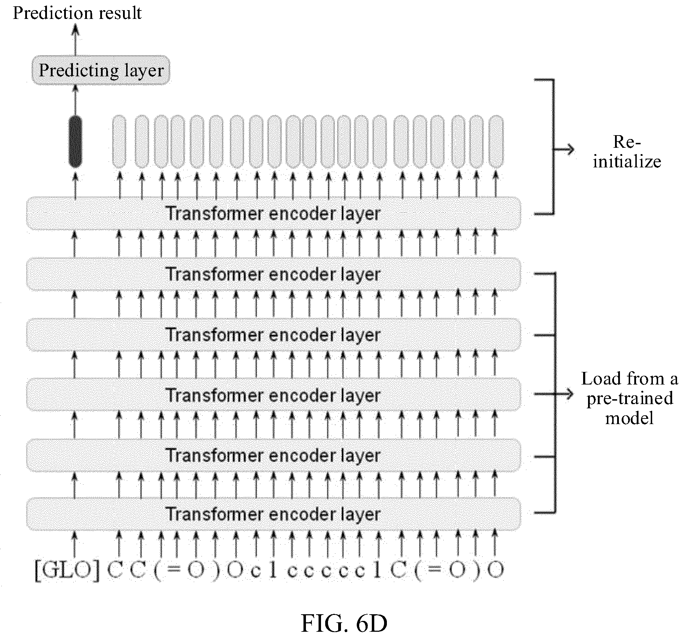

In FIG. 6D, a processing process of the K-BERT third neural network model is further described with reference to a model structure of the K-BERT' third neural network model.

The K-BERT-based third neural network model constructed based on the SMILES string-based molecular representation includes a plurality of transformer encoder layers (using six transformer encoder layers as an example herein). A word vector corresponding to the SMILES string-based molecular representation is input into the K-BERT-based third neural network model, to obtain a third molecular property prediction result (that is, a prediction result classified by the K-BERT-EDL classifier) processed by the K-BERT-based third neural network model. As shown in FIG. 6D, a dark-colored part in the figure represents a molecular feature, and a light-colored part represents a feature of another structure. In the process of the molecular feature prediction, only a prediction result of the dark-colored part in the figure is concerned.

Compared with the example shown in FIG. 6D, during implementation, atomic feature prediction, molecular feature prediction, maximizing similarity between different SMILES strings of a same molecule, and minimizing similarity between different molecules may be regarded as training targets to train the neural network model to obtain a model used for molecule prediction. For a specific molecular property prediction scenario (for example, a cardiotoxicity prediction scenario), a previously trained model used for molecule prediction may be directly loaded, to reinitialize parameters of a last layer or a plurality of neural network layers of the neural network. Then, learning continues to be performed based on training set data provided by the specific molecular property prediction scenario, to slight adjust the parameters of the last layer or the plurality of neural network layers, so that a third neural network model for the specific molecular property prediction scenario is obtained.

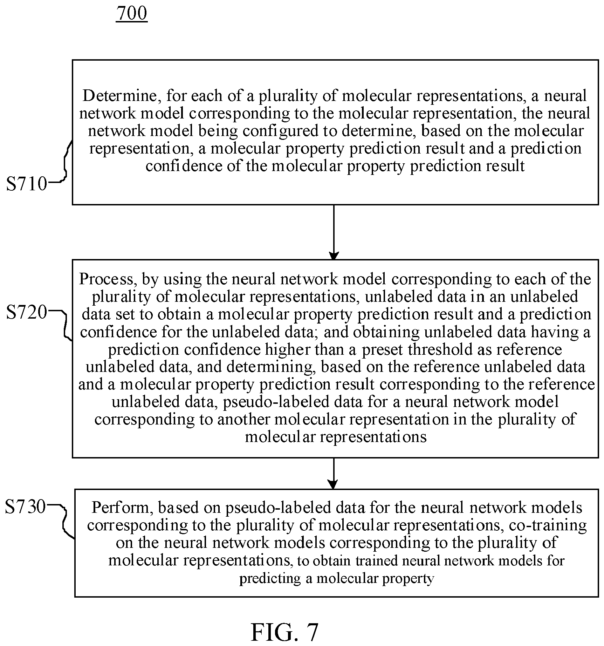

FIG. 7 is a schematic flowchart 700 of a model co-training method according to an embodiment of the present disclosure. The method is executed by a computer device.

Step S710: Determine, for each of a plurality of molecular representations, a neural network model corresponding to the molecular representation, the neural network model being configured to determine, based on the molecular representation, a molecular property prediction result and a prediction confidence of the molecular property prediction result.

In some embodiments, for each molecular representation, a plurality of neural network models corresponding to the molecular representation may be constructed based on different random seeds. In this way, variety of the neural network models can be increased, so that prediction accuracy of the neural network models corresponding to the plurality of molecular representations is improved.

Step S720: Process, by using the neural network model corresponding to each of the plurality of molecular representations, unlabeled data in an unlabeled data set to obtain a molecular property prediction result and a prediction confidence for the unlabeled data; and obtaining unlabeled data having a prediction confidence higher than a preset threshold as reference unlabeled data, and determining, based on the reference unlabeled data and a molecular property prediction result corresponding to the reference unlabeled data, pseudo-labeled data for a neural network model corresponding to another molecular representation in the plurality of molecular representations.

In some embodiments, a neural network model corresponding to each molecular representation may evaluate, based on EDL, the prediction confidence of the molecular property prediction result determined by the neural network model, to be specific, to enable each neural network model outputs a molecular property prediction result and prediction confidence corresponding to the molecular property prediction result during the training, to better evaluate the molecular property prediction result, and provide a basis for co-training of the plurality of neural network models. Therefore, each piece of unlabeled data in the unlabeled data set may be classified based on the preset threshold. Unlabeled data having corresponding prediction confidence greater than or equal to the preset threshold is regarded as data having high confidence, and unlabeled data having corresponding prediction confidence less than the preset threshold is regarded as data having low confidence.

An example in which the plurality of molecular representations include a molecular descriptor-based molecular representation, a molecular graph-based molecular representation, and a SMILES string-based molecular representation, and the neural network models corresponding to the plurality of molecular representations include a DNN-based first neural network model (corresponding to the molecular descriptor-based molecular representation), an RGCN-based second neural network model (corresponding to the molecular graph-based molecular representation), and a K-BERT-based third neural network model (corresponding to the SMILES string-based molecular representation) is as an example. When pseudo-labeled data is selected for the first neural network model, reference unlabeled data in the pseudo-labeled data may satisfy the following conditions: molecular property prediction results determined by the second neural network model and the third neural network model for the unlabeled data are the same; both; prediction confidence determined by the second neural network model and the third neural network model for the unlabeled data are high; and prediction confidence determine by the first neural network model for the unlabeled data is low. Similarly, when pseudo-labeled data is selected for the second neural network model and the third neural network model, a similar manner may be applied.

In some embodiments, for each neural network model, a training set corresponding to the neural network model is constructed, to enable data having a predetermined property in the training set and data without the predetermined property in the training set to satisfy a preset ratio (for example, for a toxicity prediction problem, data of molecules having toxicity in the training set and data of molecules without toxicity in the training set may be set to satisfy a preset ratio 1: 1). In this way, an imbalance between data having a predetermined property and data without the predetermined property can be avoided, thereby avoiding a case that there are great limitations in training set data.

In addition, for the training set corresponding to each neural network model, a proportion of pseudo-labeled data for the neural network model may be determined, and the proportion does not exceed a preset proportion threshold (for example, the proportion of the pseudo-labeled data in the training set may be set to not exceeding 15%). An objective of setting in this way is to prevent the pseudo-labeled data from occupying an excessively large proportion in the training set of the neural network model to affect a training effect of the neural network model.

In addition, for each neural network model, a most uncertain molecule in the neural network model may be added into the training set corresponding to the neural network model. The most uncertain molecule in the neural network model herein refers to a molecule that has low corresponding prediction confidence and that is determined by the neural network model. For the neural network model, this type of molecules has a large amount of information. Adding the molecule in the training set corresponding to the neural network model is conducive to improving prediction precision of the neural network model.

Step S730: Perform, based on pseudo-labeled data for the neural network models corresponding to the plurality of molecular representations, co-training on the neural network models corresponding to the plurality of molecular representations, to obtain trained neural network models for predicting a molecular property.

During a training process, a threshold may be set for a number of iterations of the co-training, so that when the number of iterations reaches the threshold, it is determined that the co-training is completed, and the neural network model at this time is determined as a trained neural network model.

According to the embodiments of the present disclosure, the plurality of molecular representations may include: a molecular descriptor-based molecular representation, a molecular graph-based molecular representation, and a SMILES string-based molecular representation. In this case, for the molecular descriptor-based molecular representation, a DNN-based first neural network model may be constructed. For the molecular graph-based molecular representation, an RGCN-based second neural network model may be constructed. For the SMILES string-based molecular representation, a K-BERT-based third neural network model may be constructed.

For the first neural network model, the first neural network model includes a plurality of FC layers. Performing co-training on the first neural network model specifically includes: determining, for pseudo-labeled data corresponding to the first neural network model, a molecular descriptor-based molecular feature vector corresponding to unlabeled data in the first neural network model, and processing the molecular feature vector via the plurality of FC layers in the first neural network model, to obtain a first molecular property prediction result; and training the first neural network model according to the first molecular property prediction result and a molecular property prediction result in the pseudo-labeled data, to adjust a model parameter of the first neural network model.

For the second neural network model, the second evidential neural network model includes a plurality of RGCN layers and a plurality of FC layers. Performing co-training on the second neural network model specifically includes: determining, for pseudo-labeled data corresponding to the second neural network model, a molecular graph corresponding to unlabeled data in the second neural network model, and processing the molecular graph via the plurality of RGCN layers in the second neural network model, to obtain feature vectors of atoms in the molecular graph, a feature vector of an atom being iteratively determined based on feature vectors of surrounding atoms; determining, based on the feature vectors of the atoms and weights corresponding to the feature vectors of the atoms, a molecular feature vector corresponding to the molecular graph; processing the molecular feature vector via the plurality of FC layers in the second neural network model, to obtain a second molecular property prediction result; and training the second neural network model according to the second molecular property prediction result and a molecular property prediction result in the pseudo-labeled data, to adjust a model parameter of the second neural network model.

For the third neural network model, the third neural network model includes a plurality of transformer encoder layers. Performing co-training on the third neural network model specifically includes: determining, for pseudo-labeled data corresponding to the third neural network model, a SMILES string corresponding to unlabeled data in the third neural network model, and processing the SMILES string via the plurality of transformer encoder layers in the third neural network model, to obtain a third molecular property prediction result; and training the third neural network model according to the third molecular property prediction result and a molecular property prediction result in the pseudo-labeled data, to adjust a model parameter of the third neural network model. The third neural network model is determined based on one or more of the following training targets: atomic feature prediction, molecular feature prediction, maximizing similarity between different SMILES strings of a same molecule, and minimizing similarity between different molecules.

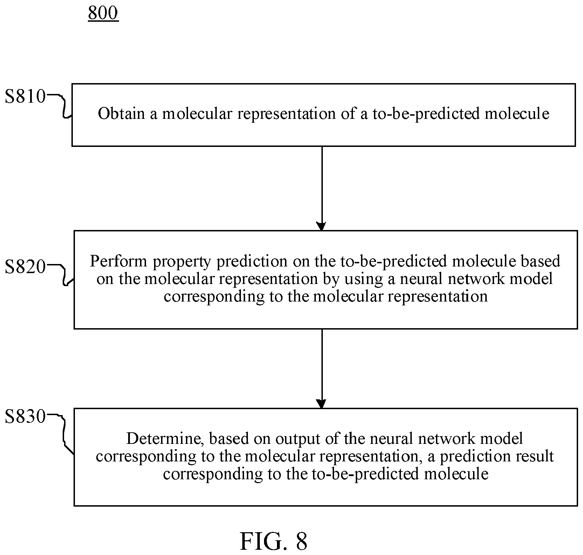

FIG. 8 is a schematic flowchart 800 of a molecular property predicting method according to an embodiment of the present disclosure. The method is executed by a computer device.

Step S810: Obtain a molecular representation of a to-be-predicted molecule.

The obtained molecular representation of the to-be-predicted molecule may be a molecular representation, or a plurality of molecular representations. In other words, molecular property prediction may be performed based on a molecular representation or a plurality of molecular representations.

Step S820: Perform property prediction on the to-be-predicted molecule based on the molecular representation by using a neural network model corresponding to the molecular representation.

According to the model training method shown in FIG. 7, training may be performed to obtain a group of neural network model including neural network models corresponding to a plurality of molecular representations. In actual application, a neural network model in the group of neural network models may be used to perform property prediction on the to-be-predicted molecule, or a plurality of neural network models in the group of neural network models may be used to perform property prediction on the to-be-predicted molecule.

Step S830: Determine, based on output of the neural network model corresponding to the molecular representation, a prediction result corresponding to the to-be-predicted molecule.

In some embodiments, a molecular property prediction result of the to-be-predicted molecule may be output based on a neural network model in a group of neural network models, or a molecular property prediction result of the to-be-predicted molecule may be determined based on an integration of output results of a plurality of neural network models in a group of neural network models.

In the molecular property predicting method based on the neural network model, a group of neural network models includes the neural network models corresponding to the plurality of molecular representations. In addition, a neural network model corresponding to each molecular representation is trained based on pseudo-labeled data corresponding to the neural network model. The pseudo-labeled data corresponding to the neural network model includes reference unlabeled data and a molecular property prediction result corresponding to the reference unlabeled data. The reference unlabeled data is unlabeled data that is in an unlabeled data set and that has corresponding prediction confidence higher than a preset threshold. The prediction confidence and the molecular property prediction result corresponding to the unlabeled data are determined by using a neural network model corresponding to another molecular representation.

In addition, prediction confidence of the neural network model may be evaluated based on the EDL, to enable the neural network model to output a molecular property prediction result and prediction confidence corresponding to the molecular property prediction result during the training.

In a group of neural network models, each molecular representation may correspond to a plurality of neural network models, and the plurality of neural network models may be constructed according to different random seeds.

According to the embodiments of the present disclosure, in a case that the plurality of molecular representations include a molecular descriptor-based molecular representation, a molecular graph-based molecular representation, and a SMILES string-based molecular representation, a neural network model corresponding to the molecular descriptor-based molecular representation may be constructed based on a DNN; a neural network model corresponding to the molecular graph-based molecular representation may be constructed based on an RGCN; and a neural network model corresponding to the SMILES string-based molecular representation may be constructed based on a K-BERT.

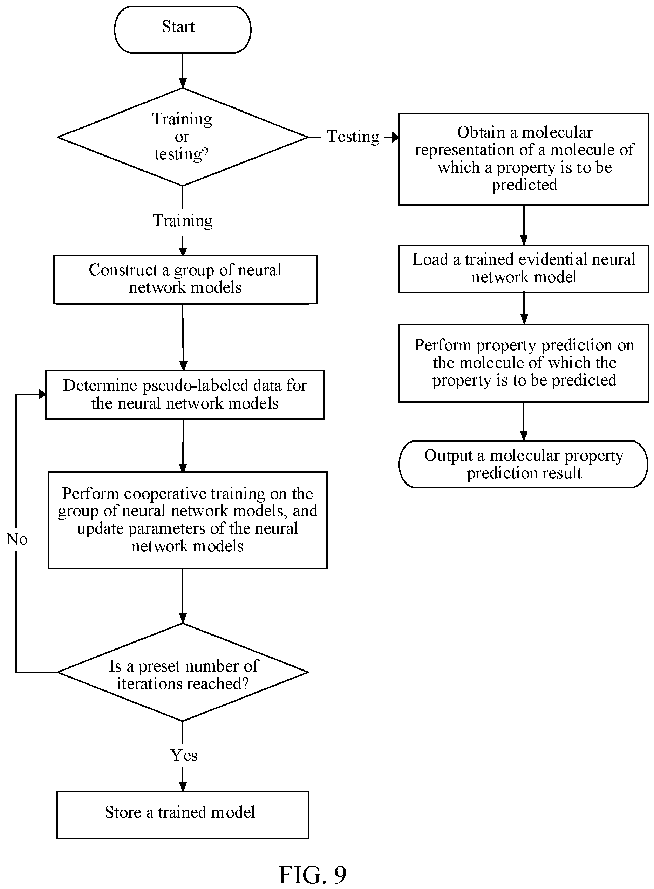

As shown in FIG. 9, a processing process based on a neural network model according to an embodiment of the present disclosure includes a training stage and a testing stage of a neural network model.

In the training stage of the neural network model, for each molecular representation in a plurality of molecular representations, one or more neural network models may be constructed, to use neural network models corresponding to the plurality of molecular representations to form a group of neural network models. Each neural network model is configured to predict a molecular property. In each iteration process during the training, for each molecular representation, a molecular property prediction result and prediction confidence corresponding to unlabeled data in an unlabeled data set are determined by using the neural network model corresponding to the molecular representation. Unlabeled data having corresponding prediction confidence higher than a preset threshold is obtained as reference unlabeled data. Pseudo-labeled data for a neural network model corresponding to another molecular representation is determined based on the reference unlabeled data and a molecular property prediction result corresponding to the reference unlabeled data. Then, neural network training is performed on pseudo-labeled data corresponding to the neural network models, and parameters of the neural network models are updated, to achieve an effect of performing co-training on a group of neural network models. If a preset number of iterations is reached during the training, a group of trained neural network models is stored. If a preset number of iterations is not reached, a group of neural network models continues to be trained.

Herein, using a criterion, in which the preset number of iterations is reached, to determine whether the co-training of the group of neural network models is completed aims to prevent over-fitting of a network or occurrence of cases that training time is too long or an obvious network optimization effect is not presented. In some embodiments, a prediction effect of a group of neural network models may be tested in real time, and then whether an optimized neural network model satisfies accuracy requirements of molecular prediction is determined. If yes, stop continuing to use an evidential neural network model.

In the testing stage of the neural network model, a molecular representation of a to-be-predicted molecule is input into a computer. The computer loads A trained neural network model, and perform forward calculation by using the neural network model to obtain a prediction result of the to-be-predicted molecule (including a molecular property prediction result, prediction confidence corresponding to the molecular property prediction result, and the like). The prediction result of the to-be-predicted molecule may be output by the computer as reference for a user.

Similarly, in an actual process of predicting the molecular property (that is, a using process of the neural network model), a processing process of the computer is similar to the testing stage, and details are not described again.

Through testing and verification, a molecular property prediction effect of the neural network model obtained by the co-training is obviously improved compared with a molecular property predicting effect of a single neural network model without co-training.

In an embodiment, the neural network model obtained by co-training is used for predicting cardiotoxicity of a drug molecule. Prediction accuracy of six neural networks shown in FIG. 3 in cardiotoxicity prediction is used as an example. As shown in Table 1, prediction accuracy of an RGCN-based RGCN-EDL classifier 1 (that is, RGCN-1) is 0.637 when co-training is performed, and the prediction accuracy is 0.616 when no co-training is performed. The accuracy is relatively improved by 3.41% (as shown in the first row of Table 1). Prediction accuracy of an RGCN-based RGCN-EDL classifier 2 (that is, RGCN-2) is 0.641 when co-training is performed, and the prediction accuracy is 0.605 when no co-training is performed. The accuracy is relatively improved by 5.95% (as shown in the second row of Table 1). Prediction accuracy of a DNN-based DNN-EDL classifier 1 (that is, DNN-1) is 0.641 when co-training is performed, and the prediction accuracy is 0.621 when no co-training is performed. The accuracy is relatively improved by 3.12% (as shown in the third row of Table 1). Prediction accuracy of a DNN-based DNN-EDL classifier 2 (that is, DNN-2) is 0.641 when co-training is performed, and the prediction accuracy is 0.614 when no co-training is performed. The accuracy is relatively improved by 4.40% (as shown in the fourth row of Table 1). Prediction accuracy of a K-BERT-based K-BERT-EDL classifier 1 (that is, K-BERT-1) is 0.650 when co-training is performed, and the prediction accuracy is 0.605 when no co-training is performed. The accuracy is relatively improved by 7.44% (as shown in the fifth row of Table 1). Prediction accuracy of a K-BERT-based K-BERT-EDL classifier 2 (that is, K-BERT-2) is 0.629 when co-training is performed, and the prediction accuracy is 0.591 when no co-training is performed. The accuracy is relatively improved by 6.43% (as shown in row 6 of Table 1). A comprehensive evaluation is performed on the results of the first six rows in Table 1 and it can be learned that comprehensive prediction accuracy of the neural networks is 0.645 when co-training is performed, and the comprehensive prediction accuracy of the neural networks is 0.622 when no co-training is performed. The accuracy is relatively improved by 3.70% (as shown in the seventh row of Table 1). It can be learned from Table 1 that by using the quasi-co-training strategy of the present invention, performance of various models is improved.

Table 1 Model predicting performance on cardiotoxicity

|  | Without co-training | With co-training | Relative improvement |
| --- | --- | --- | --- |
| RGCN-1 | 0.616 | **0.637** | 3.41% |
| RGCN-2 | 0.605 | **0.641** | 5.95% |
| DNN-1 | 0.621 | **0.641** | 3.12% |
| DNN-2 | 0.614 | **0.641** | 4.40% |
| K-BERT-1 | 0.605 | **0.650** | 7.44% |
| K-BERT-2 | 0.591 | **0.629** | 6.43% |
| Consensus | 0.622 | **0.645** | 3.70% |

In addition, 167 FDA-approved drug molecules from 2012 to 2018 (none of which are present in a training set of the neural network model) are tested, and the neural network model on which the co-training is performed in the present disclosure is compared with latest cardiotoxicity predicting models in recent years (that is, an hERG-MI, model in 2020, a DeepHIT model in 2020, a CardPred model in 2018, an OCHEM consensus 1 model in 2017, an OCHEM consensus2 model in 2017, a Pred-hERG 4.2 model in 2015 in Table 2), and results are shown in Table 2. It can be learned from Table 2 that the model of the present disclosure is optimal in terms of prediction accuracy, balanced-accuracy, and a Matthews correlation coefficient (MCC). The above results fully show effectiveness of the neural network model co-training method of the present disclosure.

Table 2 Performance on 167 FDA-approved drug molecules

|  | Accuracy | Balanced-Accuracy | MCC |
|---|---|---|---|
| Model in the present disclosure | **0.814** | **0.746** | **0.504** |
| hERG-ML (2020) | 0.790 | 0.639 | 0.373 |
| DeepHIT (2020) | 0.701 | 0.662 | 0.298 |
| CardPred (2018) | 0.756 | 0.630 | 0.317 |
| OCHEM consensus1 (2017) | 0.754 | 0.531 | 0.176 |
| OCHEM consensus2 (2017) | 0.790 | 0.608 | 0.366 |
| Pred-hERG 4.2 (2015) | 0.596 | 0.667 | 0.296 |

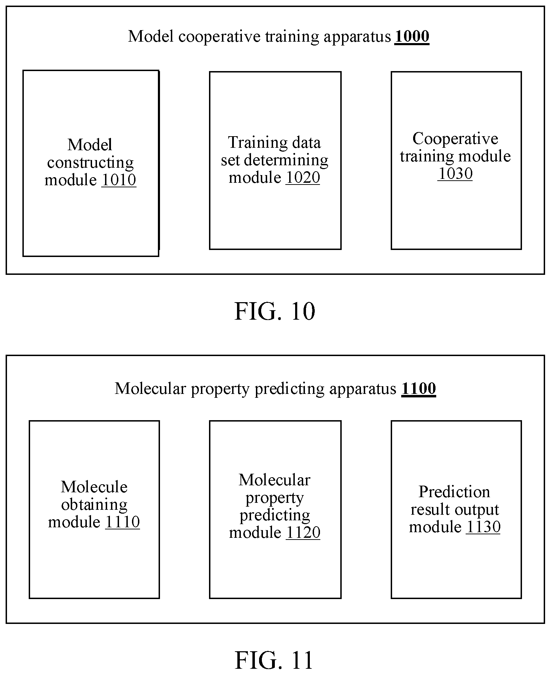

FIG. 10 is a schematic composition diagram of a model co-training apparatus according to an embodiment of the present disclosure.

According to the embodiments of the present disclosure, the model co-training apparatus 1000 may include: A model constructing module 1010, a training data set determining module 1020, and a co-training module 1030.

The model constructing module 1010 may be configured to: determine, for each of a plurality of molecular representations, a neural network model corresponding to the molecular representation, the neural network model being configured to determine, based on the molecular representation, a molecular property prediction result and a prediction confidence of the molecular property prediction result.

In some embodiments, the model constructing module 1010 may construct, for each molecular representation in the plurality of molecular representations based on different random seeds, a plurality of neural network models corresponding to the molecular representation.

The training data set determining module 1020 may be further configured to: process, by using the neural network model corresponding to each of the plurality of molecular representations, unlabeled data in an unlabeled data set to obtain a molecular property prediction result and a prediction confidence for the unlabeled data; and obtaining unlabeled data having a prediction confidence higher than a preset threshold as reference unlabeled data, and determining, based on the reference unlabeled data and a molecular property prediction result corresponding to the reference unlabeled data, pseudo-labeled data for a neural network model corresponding to another molecular representation in the plurality of molecular representations.

In some embodiments, the neural network model corresponding to each molecular representation is configured to evaluate, based on an evidential deep learning EDL method, the prediction confidence of the molecular property prediction result determined by using the neural network model.

In some embodiments, the training data set determining module 1020 may be further configured to: construct, for each neural network model, a training set corresponding to the neural network model, in the training set, a ratio of data having a predetermined property to data without the redetermined property satisfying a preset ratio, and a proportion of the pseudo-labeled data for the neural network model in the training set not exceeding a preset proportion threshold.

The co-training module 1030 may be further configured to: perform, based on pseudo-labeled data for the neural network models corresponding to the plurality of molecular representations, co-training on the neural network models corresponding to the plurality of molecular representations.

In some embodiments, the training data set determining module 1020 may be further configured to: perform, based on the training sets of the neural network models corresponding to the plurality of molecular representations, the co-training on the neural network models corresponding to the plurality of molecular representations.

According to the embodiments of the present disclosure, the plurality of molecular representations may include: a molecular descriptor-based molecular representation, a molecular graph-based molecular representation, and a SMILES string-based molecular representation. In this case, for the molecular descriptor-based molecular representation, a DNN-based first neural network model may be constructed. For the molecular graph-based molecular representation, an RGCN-based second neural network model may be constructed. For the SMILES string-based molecular representation, a K-BERT-based third neural network model may be constructed.

FIG. 11 is a schematic composition diagram of a molecular property predicting apparatus 1100 according to an embodiment of the present disclosure.

According to the embodiments of the present disclosure, the molecular property predicting apparatus 1100 based on an evidential neural network model may include: a molecule obtaining module 1110, a molecular property predicting module 1120, and a prediction result output module 1130.

The molecule obtaining module 1110 may be configured to: obtain a molecular representation of a to-be-predicted molecule.

The molecular property predicting module 1120 may be configured to: perform property prediction on the to-be-predicted molecule based on the molecular representation by using a neural network model corresponding to the molecular representation.

The prediction result output module 1130 may be configured to: determine, based on output of the neural network model corresponding to the molecular representation, a prediction result corresponding to the to-be-predicted molecule,

the neural network model corresponding to the molecular representation being trained based on pseudo-labeled data corresponding to the neural network model, the pseudo-labeled data corresponding to the neural network model comprising reference unlabeled data and a molecular property prediction result corresponding to the reference unlabeled data, the reference unlabeled data being unlabeled data that is in an unlabeled data set and that has corresponding prediction confidence higher than a preset threshold, and the prediction confidence and the molecular property prediction result corresponding to the unlabeled data being determined by using a neural network model corresponding to another molecular representation.

In general, exemplary embodiments of the present disclosure may be implemented in hardware or a private circuit, software, firmware, logic, or any combination thereof. Some aspects may be implemented in the hardware, and other aspects may be implemented in firmware or software that may is executed by a controller, a microprocessor, or another computing device. When aspects of the embodiments of the present disclosure are illustrated or described as block diagrams, flowcharts, or represented by using certain other graphical representations, it is understood that blocks, apparatuses, systems, techniques, or methods described herein may be implemented as non-limiting examples in hardware, software, firmware, a private circuit or logic, general hardware, a controller, or another computing device, or some combination thereof.

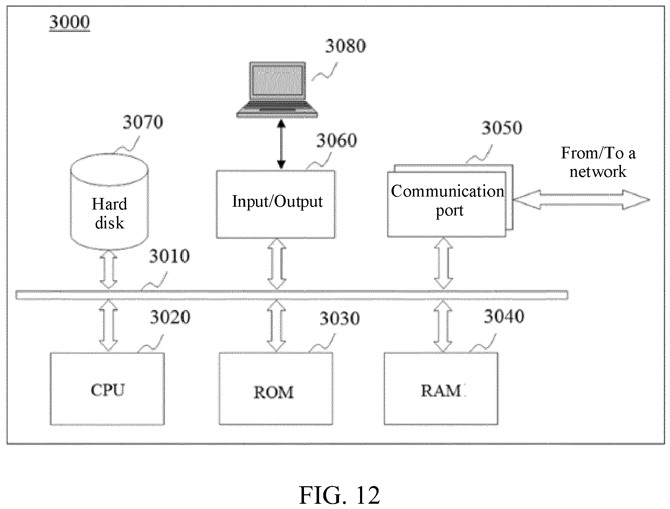

For example, the method or the apparatus according to the embodiments of the present disclosure may be implemented by using an architecture of a computing device 3000 shown in FIG. 12. As shown in FIG. 12, the computing device 3000 may include a bus 3010, one or more CPUs 3020, a read-only memory (ROM) 3030, a random access memory (RAM) 3040, a communication port 3050 connected to a network, an input/output component 3060, a hard disk 3070, and the like. A storage device in the computing device 3000, such as the ROM 3030 or the hard disk 3070, may store various data or files used in processing and/or communication of the method provided in the present disclosure and program instructions executed by the CPU. The computing device 3000 further includes a user interface 3080. Certainly, the architecture shown in FIG. 12 is only used as an example, when different devices are implemented, one or more components of the computing device shown in FIG. 12 may be omitted according to actual needs.

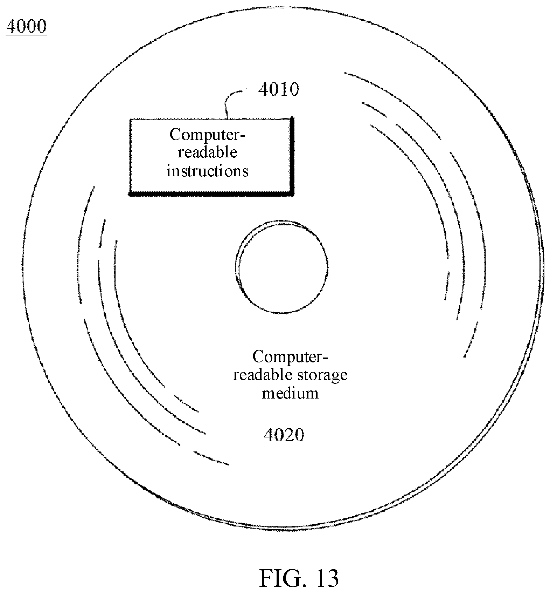

According to another aspect of the embodiments of the present disclosure, a computer-readable storage medium is further provided. FIG. 13 is a schematic diagram 4000 of a storage medium according to the present disclosure.

As shown in FIG. 13, a computer storage medium 4020 has a computer-readable instruction 4010 stored thereon. The computer-readable instruction 4010, when executed by a processor, may perform the method described with reference to the foregoing accompanying drawings according to the embodiments of the present disclosure. The computer-readable storage medium in the embodiments of the present disclosure may be a volatile memory or a non-volatile memory, or may include both volatile and non-volatile memories. The non-volatile memory may be a read-only memory (ROM), a programmable read-only memory (PROM), an erasable programmable read-only memory (EPROM), an electrically erasable programmable read-only memory (EEPROM), or a flash memory. The volatile memory may be a random access memory (RAM) used as an external cache. Through illustrative but not limited description, many forms of RAMs may be used, for example, a static random access memory (SRAM), a dynamic random access memory (DRAM), a synchronous dynamic random access memory (SDRAM), a double data rate synchronous dynamic random access memory (DDR SDRAM), an enhanced synchronous dynamic random access memory (ESDRAM), a synchronous link dynamic random access memory (SLDRAM), and a direct rambus random access memory (DR RAM). It is to be noted that the memories of the method described herein are intended to include but are not limited to these and any other suitable types of memories.

An embodiment of the present disclosure further provides a computer program product or a computer program, including computer instructions stored in a computer-readable storage medium. A processor of a computer device reads the computer instructions from the computer-readable storage medium, and the processor executes the computer instructions, so that the computer device performs the method according to the embodiments of the present disclosure.

The flowcharts and block diagrams in the accompanying drawings illustrate possible system architectures, functions, and operations that may be implemented by a system, a method, and a computer program product according to various embodiments of the present disclosure. In this regard, each block in the flowcharts or block diagrams may represent a module, a program segment, or a part of code, which includes at least one executable instruction used for implementing specified logic functions. It is also to be noted that in some implementations used as substitutes, functions annotated in blocks may alternatively occur in a sequence different from those annotated in the accompanying drawings. For example, actually two blocks shown in succession may be performed basically in parallel, and sometimes the two blocks may be performed in a reverse sequence. This is determined by a related function. It is also to be noted that each block in a block diagram and/or a flowchart and a combination of blocks in the block diagram and/or the flowchart may be implemented by using a dedicated hardware-based system configured to perform a specified function or operation, or may be implemented by using a combination of dedicated hardware and a computer instruction.

Specific terms are used in the present disclosure to describe the embodiments of the present disclosure. For example, "first/second embodiment", "an embodiment", "and/or", and "some embodiments" mean a specific feature, structure, or characteristic related to at least one embodiment of the present disclosure. Therefore, it is to be emphasized and noted that "an embodiment" or "one embodiment" or "an alternative embodiment" mentioned twice or more at different places in the specification do not necessarily refer to a same embodiment. In addition, some features, structures or characteristics of one or more embodiments of the present disclosure may be combined appropriately.

Unless otherwise defined, all terms (including technical and scientific terms) used herein have the same meaning as those commonly understood by a person of ordinary skill in the art to which the present invention belongs. It is further to be understood that the terms such as those defined in commonly used dictionaries are to be interpreted as having meanings that are consistent with the meanings in the context of the related art, and are not to be interpreted in an idealized or extremely formalized sense, unless expressively so defined herein.

The above is description of the present invention, and is not to be considered as a limitation to the present invention. Although several exemplary embodiments of the present invention are described, a person skilled in the art may easily understand that many changes can be made to the exemplary embodiments without departing from novel teaching and advantages of the present invention. Therefore, the changes are intended to be included within the scope of the present invention as defined by the claims. It is to be understood that the above is description of the present invention, and is not to be considered to be limited by the disclosed specific embodiments, and modifications to the disclosed embodiments and other embodiments fall within the scope of the appended claims. The present invention is subject to the claims and equivalents thereof.

**Claims**

1. A model co-training method, executable by a computer device, the method comprising:

   determining, for each of a plurality of molecular representations, a neural network model corresponding to the molecular representation, the neural network model being configured to determine, based on the molecular representation, a molecular property prediction result and a prediction confidence of the molecular property prediction result;
   processing, by using the neural network model corresponding to each of the plurality of molecular representations, unlabeled data in an unlabeled data set to obtain a molecular property prediction result and a prediction confidence for the unlabeled data; and obtaining unlabeled data having a prediction confidence higher than a preset threshold as reference unlabeled data, and determining, based on the reference unlabeled data and a molecular property prediction result corresponding to the reference unlabeled data, pseudo-labeled data for a neural network model corresponding to another molecular representation in the plurality of molecular representations; and
   performing, based on pseudo-labeled data for neural network models corresponding to the plurality of molecular representations, co-training on the neural network models corresponding to the plurality of molecular representations, to obtain trained neural network models for predicting a molecular property.

2. The method according to claim 1, wherein the neural network model corresponding to each of the plurality of molecular representations is configured to determine, by using an evidential deep learning, EDL, method, the prediction confidence of the molecular property prediction result.

3. The method according to claim 1, further comprising:

   constructing, for each of the plurality of neural network models, a training set corresponding to the neural network model, wherein in the training set, a ratio of data having a predetermined property to data without the predetermined property satisfies a preset ratio, and a proportion of the pseudo-labeled data for the neural network model in the training set does not exceeding a preset proportion threshold; and

the performing, based on pseudo-labeled data for neural network models corresponding to the plurality of molecular representations, co-training on the neural network models corresponding to the plurality of molecular representations comprises:

performing, based on the training sets for the neural network models corresponding to the plurality of molecular representations, the co-training on the neural network models corresponding to the plurality of molecular representations.

4. The method according to claim 1, wherein the determining, for each of a plurality of molecular representations, a neural network model corresponding to the molecular representation comprises:
constructing, for each of the plurality of molecular representations and based on different random seeds, a plurality of neural network models corresponding to the molecular representation.

5. The method according to claim 1, wherein the plurality of molecular representations comprise: a molecular descriptor-based molecular representation, a molecular graph-based molecular representation, and a simplified molecular input line entry system SMILES string-based molecular representation; and

the determining, for each of a plurality of molecular representations, a neural network model corresponding to the molecular representation comprises:

constructing, for the molecular descriptor-based molecular representation, a first neural network model based on a deep neural network DNN;

constructing, for the molecular graph-based molecular representation, a second neural network model based on a relational graph convolution network RGCN; and

constructing, for the SMILES string-based molecular representation, a third neural network model based on a knowledge-based bidirectional encoder representation from transformers K-BERT.

6. The method according to claim 5, wherein the first neural network model comprises a plurality of fully connected FC layers; and

the performing, based on pseudo-labeled data for the neural network models corresponding to the plurality of molecular representations, co-training on the neural network models corresponding to the plurality of molecular representations comprises:

determining, for pseudo-labeled data corresponding to the first neural network model, a molecular descriptor-based molecular feature vector corresponding to unlabeled data in the first neural network model, and processing the molecular feature vector by using the plurality of FC layers in the first neural network model, to obtain a first molecular property prediction result; and

training the first neural network model by using the first molecular property prediction result and a molecular property prediction result in the pseudo-labeled data.

7. The method according to claim 5, wherein the second neural network model comprises a plurality of RGCN layers and a plurality of FC layers; and

the performing, based on pseudo-labeled data for the neural network models corresponding to the plurality of molecular representations, co-training on the neural network models corresponding to the plurality of molecular representations comprises:

determining, for pseudo-labeled data corresponding to the second neural network model, a molecular graph corresponding to unlabeled data in the second neural network model, and processing the molecular graph by using the plurality of RGCN layers in the second neural network model, to obtain feature vectors of atoms in the molecular graph, the feature vectors of the atoms being iteratively determined based on feature vectors of surrounding atoms; determining, based on the feature vectors of the atoms and weights corresponding to the feature vectors of the atoms, a molecular feature vector corresponding to the molecular graph; and processing the molecular feature vector via the plurality of FC layers in the second neural network model, to obtain a second molecular property prediction result; and

training the second neural network model according to the second molecular property prediction result and a molecular property prediction result in the pseudo-labeled data.

8. The method according to claim 5, wherein the third neural network model comprises a plurality of transformer encoder layers; and

the performing, based on pseudo-labeled data for the neural network models corresponding to the plurality of molecular representations, co-training on the neural network models corresponding to the plurality of molecular representations comprises:

determining, for pseudo-labeled data corresponding to the third neural network model, a SMILES string corresponding to unlabeled data in the third neural network model, and processing the SMILES string by using the plurality of transformer encoder layers in the third neural network model, to obtain a third molecular property prediction result; and

training the third neural network model by using the third molecular property prediction result and a molecular property prediction result in the pseudo-labeled data, the third neural network model being determined based on one or more of the following training targets: atomic feature prediction, molecular feature prediction, maximizing similarity between different SMILES strings of a same molecule, and minimizing similarity between different molecules.

9. A molecular property predicting method, executable a computer device, the method comprising:

obtaining a molecular representation of a to-be-predicted molecule;

performing property prediction on the to-be-predicted molecule based on the molecular representation by using a neural network model corresponding to the molecular representation; and

determining, based on output of the neural network model corresponding to the molecular representation, a prediction result corresponding to the to-be-predicted molecule,

the neural network model corresponding to the molecular representation being trained based on pseudo-labeled data corresponding to the neural network model, the pseudo-labeled data corresponding to the neural network model comprising reference unlabeled data and a molecular property prediction result corresponding to the reference unlabeled data, the reference unlabeled data being in an unlabeled data set and having a prediction confidence higher than a preset threshold, and the prediction confidence and the molecular property prediction result corresponding to the unlabeled data being determined by using a neural network model corresponding to another molecular representation.

10. The method according to claim 9, wherein
each molecular representation corresponds to a plurality of neural network models, and the plurality of neural network models are constructed according to different random seeds.

11. The method according to claim 9, wherein the plurality of molecular representations comprise: a molecular descriptor-based molecular representation, a molecular graph-based molecular representation, and a simplified molecular input line entry system SMILES string-based molecular representation,

a neural network model corresponding to the molecular descriptor-based molecular representation being constructed based on a deep neural network DNN;

a neural network model corresponding to the molecular graph-based molecular representation being constructed based on a relational graph convolution network RGCN; and

a neural network model corresponding to the SMILES string-based molecular representation being constructed based on a knowledge-based bidirectional encoder representation from transformers K-BERT.

12. A model co-training apparatus, comprising:

a model constructing module, configured to determine, for each of a plurality of molecular representations, a neural network model corresponding to the molecular representation, the neural network model being configured to determine, based on the molecular representation, a molecular property prediction result and a prediction confidence of the molecular property prediction result;

a training data set determining module, configured to: process, by using the neural network model corresponding to each of the plurality of molecular representations, unlabeled data in an unlabeled data set to obtain a molecular property prediction result and a prediction confidence for the unlabeled data; and obtaining unlabeled data having a prediction confidence higher than a preset threshold as reference unlabeled data, and determining, based on the reference unlabeled data and a molecular property prediction result corresponding to the reference unlabeled data, pseudo-labeled data for a neural network model corresponding to another molecular representation in the plurality of molecular representations; and

a co-training module, configured to perform, based on pseudo-labeled data for the neural network models corresponding to the plurality of molecular representations, co-training on the neural network models corresponding to the plurality of molecular representations, to obtain trained neural network models for predicting a molecular property.

13. A molecular property predicting apparatus, comprising:

a molecule obtaining module, configured to obtain a molecular representation of a to-be-predicted molecule;
a molecular property predicting module, configured to perform, based on the molecular representation by using a neural network model corresponding to the molecular representation, property prediction on the to-be-predicted molecule; and
a prediction result output module, configured to determine, based on output of the neural network model corresponding to the molecular representation, a prediction result corresponding to the to-be-predicted molecule, the neural network model corresponding to the molecular representation being trained based on pseudo-labeled data corresponding to the neural network model, the pseudo-labeled data corresponding to the neural network model comprising reference unlabeled data and a molecular property prediction result corresponding to the reference unlabeled data, the reference unlabeled data being in an unlabeled data set and having a prediction confidence higher than a preset threshold, and the prediction confidence and the molecular property prediction result corresponding to the unlabeled data being determined by using a neural network model corresponding to another molecular representation.

14. A computer device, comprising a processor and a memory, the memory having program instructions stored therein; and
the processor being configured to execute the program instructions stored in the memory, to perform the method according to any one of claims 1 to 11.

15. A computer program product, comprising computer software code, the computer software code, when run by a processor, being used for implementing the method according to any one of claims 1 to 11.

16. A computer-readable storage medium, having computer-executable instructions stored thereon, the instructions, when executed by a processor, being user for implementing the method according to any one of claims 1 to 11.

Hit compound/
Lead compound

Absorption

Physicochemical
property

Pharmaceutical
chemistry

Toxicity

Distribution

Metabolism

Excretion

Candidate drug

FIG. 1

| apol | ast_fraglike | ... | zagreb |
|---|---|---|---|
| 24.38 | 1.00 | ... | 60.00 |

FIG. 2A

FIG. 2B

OC(C1=C(OC(C)=O)C=CC=C1)=O

FIG. 2C

EP 4 421 655 A1

Pseudo-label rule (using a molecular graph as an example)

| RGCN-EDL classifier 1 | RGCN-EDL classifier 2 | DNN-EDL classifier 1 | DNN-EDL classifier 2 | K-BERT-EDL classifier 1 | K-BERT-EDL classifier 2 |

High uncertainty     Low uncertainty     Consistent prediction → Pseudo-labeled data of a molecular graph model

Multi-perspectives

Initial training set data

Molecular graph data

Pseudo-labeled data of a molecular graph model

RGCN-EDL classifier 1

RGCN-EDL classifier 2

Molecular descriptor data

Pseudo-labeled data of a molecular descriptor model

DNN-EDL classifier 1

DNN-EDL classifier 2

SMILE string data

Pseudo-labeled data of a SMILE string model

K-BERT-EDL classifier 1

K-BERT-EDL classifier 2

Unlabeled data

FIG. 3

| apol | ast_fraglike | ... | zagreb |
|------|--------------|-----|--------|
| 24.38 | 1.00 | ... | 60.00 |

O(C(=O)c1ccccc1C(O)=O

Molecular descriptor

FC1  FC2  FC3

Classification target

Output

FIG. 4

FIG. 5

Predict an atomic
property

FC layer

Atom
embedding

BERT

[GLO] C C ( = O ) O c 1 c c c c c 1 C ( = O ) O

FIG. 6A

Predict a molecular
property

FC layer

Global
embedding

BERT

[GLO] C C ( = O ) O c 1 c c c c c 1 C ( = O ) O

FIG. 6B

Canonical SMILES 1   Canonical SMILES 2   Canonical SMILES 32

**BERT**

Canonical SMILES 1 embedding   Canonical SMILES 2 embedding   Canonical SMILES 32 embedding

Minimize similarity

Maximize similarity   Maximize similarity   Maximize similarity

FIG. 6C

Prediction result

Predicting layer

Transformer encoder layer

Re-initialize

Transformer encoder layer

Transformer encoder layer

Transformer encoder layer

Load from a pre-trained model

Transformer encoder layer

Transformer encoder layer

[GLO] C C ( = O ) O c 1 c c c c 1 C ( = O ) O

FIG. 6D

700

S710 — Determine, for each of a plurality of molecular representations, a neural network model corresponding to the molecular representation, the neural network model being configured to determine, based on the molecular representation, a molecular property prediction result and a prediction confidence of the molecular property prediction result

S720 — Process, by using the neural network model corresponding to each of the plurality of molecular representations, unlabeled data in an unlabeled data set to obtain a molecular property prediction result and a prediction confidence for the unlabeled data; and obtaining unlabeled data having a prediction confidence higher than a preset threshold as reference unlabeled data, and determining, based on the reference unlabeled data and a molecular property prediction result corresponding to the reference unlabeled data, pseudo-labeled data for a neural network model corresponding to another molecular representation in the plurality of molecular representations

S730 — Perform, based on pseudo-labeled data for the neural network models corresponding to the plurality of molecular representations, co-training on the neural network models corresponding to the plurality of molecular representations, to obtain trained neural network models for predicting a molecular property

FIG. 7

800

S810 — Obtain a molecular representation of a to-be-predicted molecule

S820 — Perform property prediction on the to-be-predicted molecule based on the molecular representation by using a neural network model corresponding to the molecular representation

S830 — Determine, based on output of the neural network model corresponding to the molecular representation, a prediction result corresponding to the to-be-predicted molecule

FIG. 8

Start

Training or testing?

—— Testing → Obtain a molecular representation of a molecule of which a property is to be predicted

Training

Construct a group of neural network models

Determine pseudo-labeled data for the neural network models

Perform cooperative training on the group of neural network models, and update parameters of the neural network models

No

Is a preset number of iterations reached?

Yes

Store a trained model

Load a trained evidential neural network model

Perform property prediction on the molecule of which the property is to be predicted

Output a molecular property prediction result

FIG. 9

Model cooperative training apparatus **1000**

| Model constructing module <u>1010</u> | Training data set determining module <u>1020</u> | Cooperative training module <u>1030</u> |

**FIG. 10**

Molecular property predicting apparatus **1100**

| Molecule obtaining module <u>1110</u> | Molecular property predicting module <u>1120</u> | Prediction result output module <u>1130</u> |

**FIG. 11**

FIG. 12

FIG. 13

## INTERNATIONAL SEARCH REPORT

| International application No. |
| --- |
| **PCT/CN2023/078322** |

**A. CLASSIFICATION OF SUBJECT MATTER**

G06F 16/35(2019.01)i

According to International Patent Classification (IPC) or to both national classification and IPC

**B. FIELDS SEARCHED**

Minimum documentation searched (classification system followed by classification symbols)

IPC: G06F,G06K,G06N,G16H

Documentation searched other than minimum documentation to the extent that such documents are included in the fields searched

Electronic data base consulted during the international search (name of data base and, where practicable, search terms used)

VEN, CNTXT, WOTXT, EPTXT, USTXT, CNKI, IEEE: 分子, 化合物, 性质, 预测, 神经网络, 协同训练, 置信度, 阈值, 伪标签, Molecules, Compounds, Properties, prediction, Neural networks, cooperative training, Confidence, Threshold, Pseudo-Label

**C. DOCUMENTS CONSIDERED TO BE RELEVANT**

| Category* | Citation of document, with indication, where appropriate, of the relevant passages | Relevant to claim No. |
| --- | --- | --- |
| X | CN 113806546 A (NATIONAL UNIVERSITY OF DEFENSE TECHNOLOGY OF PLA) 17 December 2021 (2021-12-17)<br>    description, paragraphs [0043]-[0062] and [0078] | 1-16 |
| A | CN 110263824 A (ALIBABA GROUP HOLDING LIMITED) 20 September 2019 (2019-09-20)<br>    entire document | 1-16 |
| A | CN 113361627 A (UNIVERSITY OF SCIENCE AND TECHNOLOGY OF CHINA) 07 September 2021 (2021-09-07)<br>    entire document | 1-16 |
| A | WO 2021244926 A1 (KONINKLIJKE PHILIPS N. V.) 09 December 2021 (2021-12-09)<br>    entire document | 1-16 |

☐ Further documents are listed in the continuation of Box C.    ☑ See patent family annex.

| * Special categories of cited documents: | |
| --- | --- |
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **15 May 2023** | **19 May 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
**Information on patent family members**

International application No.

**PCT/CN2023/078322**

| Patent document cited in search report | | | Publication date (day/month/year) | Patent family member(s) | | | Publication date (day/month/year) |
|---|---|---|---|---|---|---|---|
| CN | 113806546 | A | 17 December 2021 | None | | | |
| CN | 110263824 | A | 20 September 2019 | None | | | |
| CN | 113361627 | A | 07 September 2021 | None | | | |
| WO | 2021244926 | A1 | 09 December 2021 | EP | 4162504 | A1 | 12 April 2023 |
| | | | | CN | 115699199 | A | 03 February 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022105584935